# EUROPEAN PATENT APPLICATION

(11) **EP 4 353 107 A1**
(43) Date of publication of application: **17.04.2024**
(21) Application number: 22804053.1
(22) Date of filing: 20.05.2022
(51) Int. Cl.: A24F 47/00

(54) **ELECTRONIC ATOMIZATION APPARATUS AND CONTROL METHOD**

(30) Priority: 21.05.2021 CN 202110560160
(71) Applicant: Shenzhen First Union Technology Co., Ltd., Shenzhen, Guangdong 518000 (CN)
(72) Inventor: LI, Xinjun, Shenzhen, Guangdong 518000 (CN); XU, Zhongli, Shenzhen, Guangdong 518000 (CN); LI, Yonghai, Shenzhen, Guangdong 518000 (CN)
(74) Representative: Proi World Intellectual Property GmbH
(86) International application number: PCT/CN2022/094011
(87) International publication number: WO 2022/242733

(57) **Abstract**

This application provides an electronic atomization apparatus and a control method. The electronic atomization apparatus includes: a liquid storage chamber; a vibrable element, configured to generate vibration to atomize a liquid substrate for generating an aerosol; and a peak voltage detection module, configured to detect a peak voltage at both terminals of the vibrable element; and a controller, configured to control vibration of the vibrable element based on the peak voltage. The foregoing electronic atomization apparatus may obtain liquid substrate supply and working parameters on the vibrable element by detecting a peak voltage at the both terminals of the vibrable element, to correspondingly adjust operation of the vibrable element to prevent the vibrable element from working when the liquid supply is inadequate or in a deviated state.

## Description

### CROSS-REFERENCE TO RELATED APPLICATIONS

This application claims priority to Chinese Patent Application 202110560160.1, filed with the China National Intellectual Property Administration on May 21, 2021 and entitled "ELECTRONIC ATOMIZATION APPARATUS AND CONTROL METHOD", which is incorporated herein by reference in its entirety.

### TECHNICAL FIELD

Embodiments of this application relate to the field of electronic atomization technologies, and particularly, to an electronic atomization apparatus and a control method.

### BACKGROUND

During use of tobacco products (for example, cigarettes and cigars), tobacco is burn to produce tobacco smoke. People are trying to manufacture products that release compounds without burning them to replace the products burning tobacco.

An example of such products is an atomization apparatus, for example, a heating atomization apparatus or an ultrasonic atomization apparatus. In the heating atomization apparatus, liquid delivered by a capillary element is heated and vaporized through a heating element, to generate an aerosol for inhalation. In the ultrasonic atomization apparatus, liquid delivered by a capillary element is dispersed into particles by a vibrating component, for example, a piezoelectric ceramic sheet, capable of vibrating at a high frequency in a reciprocating manner, to form an inhalable aerosol. The liquid may include nicotine and/or a flavor and/or an aerosol-generating substance (for example, glycerin). When the foregoing atomization apparatus works, the liquid delivered by the capillary element to the heating element or the vibrating component needs to be kept adequate, to avoid "dry burning" that occurs when the liquid supply is inadequate. The heating atomization apparatus usually determines, by monitoring a temperature of the heating element, whether the liquid supplied to the vibrating component is adequate. However, for the ultrasonic atomization apparatus, whether the liquid supplied to the vibrating component is adequate cannot be accurately determined.

### SUMMARY

This application provides an electronic atomization apparatus, including:
a liquid storage chamber, configured to store a liquid substrate;
a vibrable element, configured to generate vibration to atomize the liquid substrate for generating an aerosol;
a peak voltage detection module, configured to detect a peak voltage at both terminals of the vibrable element; and
a controller, configured to control vibration of the vibrable element based on the peak voltage.

In some embodiments, the controlling vibration of the vibrable element may include controlling a vibration frequency, an amplitude, a displacement, a speed or acceleration, and a phase of the vibrable element.

The foregoing peak voltage is a maximum of the voltage that changes simple harmonically at the both terminals of the vibrable element.

In a preferable embodiment, the controller is configured to determine, based on the peak voltage, a damping that the vibrable element is subjected to, and control vibration of the vibrable element according to the damping. In an optional embodiment, the damping that the vibrable element is subjected to is generated by the liquid substrate provided to the vibrable element.

In a preferable embodiment, the controller is configured to compare the damping with a preset value, and prevent, when the damping is less than the preset value, the vibrable element from vibrating.

In a preferable embodiment, the peak voltage detection module includes:
a holding capacitor, configured to hold the peak voltage at the both terminals of the vibrable element.

In a preferable embodiment, the peak voltage detection module further includes:
an operational amplifier, configured to output a voltage at the both terminals of the vibrable element to the holding capacitor; and
a voltage follower, configured to output the peak voltage that are at the both terminals of the vibrable element and that are held by the holding capacitor.

In a preferable embodiment, the peak voltage detection module further includes:
a discharge switch, configured to discharge the holding capacitor in an on state.

In a preferable embodiment, a sampling terminal of the operational amplifier is connected to the vibrable element.

The holding capacitor includes three channels, where a first channel is connected to an output terminal of the operational amplifier, a second channel is connected to the discharge switch, and a third channel is connected to a sampling terminal of the voltage follower.

In a preferable embodiment, the vibrable element includes at least piezoelectric ceramics.

In a preferable embodiment, the controller is configured to determine a vibration frequency of the vibrable element based on the peak voltage.

In a preferable embodiment, the controller is further configured to:
adjust the vibration frequency of the vibrable element, to keep the vibration frequency the same as or substantially close to a preset frequency.

In a preferable embodiment, the controller is configured to:
use a changing frequency to drive the vibrable element to vibrate, and detect the peak voltage at the both terminals of the vibrable element; and
determine an optimal resonant frequency of the vibrable element according to a maximum of the peak voltage.

This application further provides an electronic atomization apparatus, including:
a liquid storage chamber, configured to store a liquid substrate;
a vibrable element, configured to generate vibration to atomize the liquid substrate for generating an aerosol; and
the controller, configured to control vibration of the vibrable element according to a damping of the liquid substrate that the vibrable element is subjected to.

In a preferable embodiment, the controller is configured to compare the damping with a preset value, and prevent, when the damping is less than the preset value, the vibrable element from vibrating.

This application further provides a control method for an electronic atomization apparatus. The electronic atomization apparatus includes a liquid storage chamber, configured to store a liquid substrate; and
a vibrable element, configured to generate vibration to atomize the liquid substrate for generating an aerosol.

The method includes:
detecting a peak voltage at both terminals of the vibrable element; and
controlling vibration of the vibrable element based on the peak voltage.

In a preferable embodiment, the controlling vibration of the vibrable element based on the peak voltage includes:
determining, based on the peak voltage, a damping of the liquid substrate that the vibrable element is subjected to, and controlling vibration of the vibrable element according to the damping.

In a preferable embodiment, the controlling vibration of the vibrable element based on the peak voltage includes:
determining a vibration frequency of the vibrable element based on the peak voltage; and
adjust the vibration frequency of the vibrable element, to keep the vibration frequency the same as or substantially close to a preset frequency.

The foregoing electronic atomization apparatus may obtain liquid substrate supply and working parameters on the vibrable element by detecting a peak voltage at the both terminals of the vibrable element, to correspondingly adjust operation of the vibrable element to prevent the vibrable element from working when the liquid supply is inadequate or in a deviated state.

### BRIEF DESCRIPTION OF THE DRAWINGS

One or more embodiments are exemplarily described with reference to the corresponding figures in the accompanying drawings, and the descriptions are not to be construed as limiting the embodiments. Components in the accompanying drawings that have same reference numerals are represented as similar components, and unless otherwise particularly stated, the figures in the accompanying drawings are not drawn to scale.
FIG. 1 is a schematic structural diagram of an electronic atomization apparatus according to an embodiment of this application;
FIG. 2 is a structural block diagram of an embodiment of a circuit in FIG. 1;
FIG. 3 is a schematic diagram of basic components of an embodiment of a drive module in FIG. 2;
FIG. 4 is a schematic diagram of a voltage change of a vibrable element sampled at a first sampling point in FIG. 3;
FIG. 5 is a schematic diagram of a basic component of an embodiment of a peak voltage detection module in FIG. 2;
FIG. 6 is a schematic diagram of an output result of the peak voltage detection module in FIG. 5;
FIG. 7 is a schematic diagram of a voltage change of a vibrable element when a liquid substrate is adequate in an embodiment;
FIG. 8 is a schematic diagram of a voltage change of a vibrable element after a liquid substrate is consumed in an embodiment;
FIG. 9 is a schematic diagram of a control method for an electronic atomization apparatus in an embodiment;
FIG. 10 is a schematic diagram of a voltage change of a vibrable element during a frequency sweep in an embodiment; and
FIG. 11 is a schematic diagram of a control method for an electronic atomization apparatus in another embodiment.

### DETAILED DESCRIPTION

For ease of understanding of this application, this application is described below in more detail with reference to accompanying drawings and specific implementations.

An embodiment of this application provides an electronic atomization apparatus, configured to atomize a liquid substrate to generate an aerosol for inhalation. The electronic atomization apparatus disclosed in this application may also be represented as an aerosol generation system or a drug delivery product. Therefore, such an apparatus or system may be adapted to provide one or more substances (for example, flavors and/or pharmaceutically active ingredients) in an inhalable form or state. For example, an inhalable substance may be basically in a form of an aerosol (for example, a suspension of fine solid particles or liquid droplets in a gas).

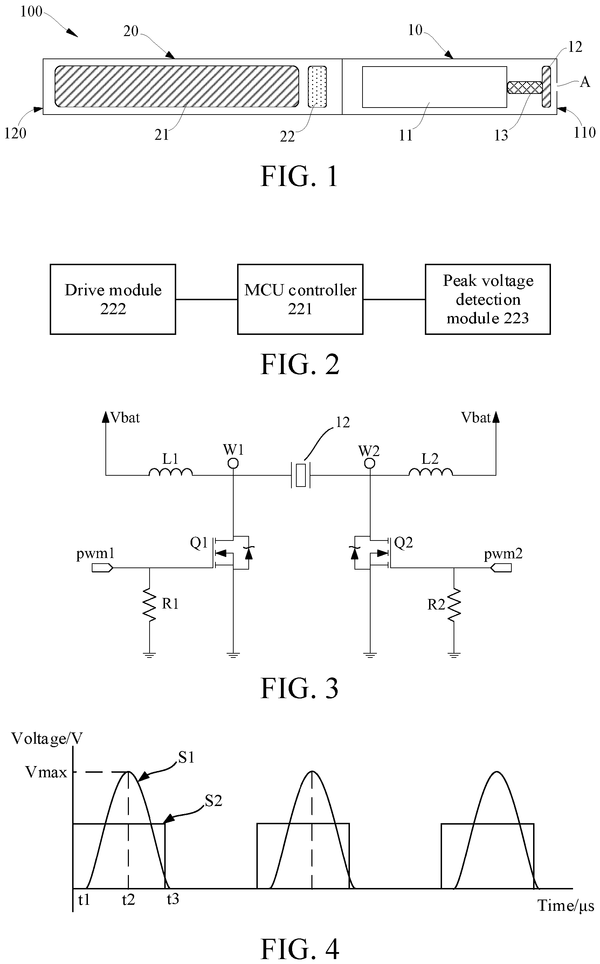

FIG. 1 is a schematic structural diagram of an embodiment of an electronic atomization apparatus 100. The apparatus usually includes several components arranged in an external body or housing (which may be referred to as a casing). An overall design of the external body or the housing is changeable, and a type or configuration of the external body that can define an overall size and shape of the electronic atomization apparatus 100 is changeable. Generally, an elongated body in a shape similar to a cigarette or cigar may be formed from a single one-piece casing, or an elongated casing may be formed from two or more separable bodies. For example, the electronic atomization apparatus 100 may have a control body at one end, where the control body has a casing including one or more reusable components (for example, a chargeable battery and/or a storage battery of a rechargeable supercapacitor, and various electronic devices configured to control operations on the product), and have, at an other end, an external body or a housing that is removably coupled and includes a disposable part (for example, a disposable flavor-including cartridge).

Specifically, further referring to FIG. 1, the electronic atomization apparatus 100 includes an atomizer 10 configured to store a liquid substrate and atomize the liquid substrate to generate an aerosol and a power supply mechanism 20 configured to supply power to the atomizer 10. The power supply mechanism 20 and the atomizer 10 are removably aligned in a functional relationship. The atomizer 10 may be connected to the power supply mechanism 20 by utilizing various mechanisms, leading to a threaded engagement, a press fit engagement, an interference fit, a magnetic engagement, or the like. In some exemplary implementations, if the atomizer 10 and the power supply mechanism 20 are in an assembled configuration, the electronic atomization apparatus 100 may be substantially in a shape of a stick, a flat cylinder, a rod, a column, or the like.

In an optional implementation, the power supply mechanism 20 and the atomizer 10 may include detached and separate casings or external bodies formed from any material in a variety of different materials. The casing may be formed from any suitable material that is structurally sound. In some examples, the casing can be formed from a metal such as stainless steel or aluminum or an alloy. Other suitable materials include various plastics (for example, polycarbonate), metal-plating over plastic, ceramics, and the like.

Further, as shown in FIG. 1, the electronic atomization apparatus 100 has a proximal end 110 and a distal end 120 that are opposite to each other along a length direction; In use, the proximal end 110 is usually used as an end vaped by a user, and the distal end 120 is used as an end far away from the user. The atomizer 10 is arranged at the proximal end 110, and the power supply mechanism 20 is arranged at the distal end 120.

According to FIG. 1, the power supply mechanism 20 includes:
a cell 21, configured for power supply, where the cell 21 may include, for example, a battery (which is disposable or rechargeable), a rechargeable supercapacitor, a rechargeable solid-state battery (SSB), a rechargeable lithium-ion battery (LiB), or the like, or a combination thereof; and
a circuit 22, configured to guide a current between the cell 21 and the atomizer 10.

According to FIG. 1, the atomizer 10 includes:
a mouthpiece opening A, located at the proximal end 110 and configured for the user to inhale;
a liquid storage chamber 11, configured to store a liquid substrate;
a vibrable element 12, in fluid communication with the liquid storage chamber 11 and configured atomize, through mechanical vibration, the liquid substrate delivered to the vibrable element 12 into an aerosol; and
a liquid delivery element 13, configured to deliver the liquid substrate between the liquid storage chamber 11 and the vibrable element 12.

In an optional implementation, the vibrable element 12 may be a common sheet-shaped ultrasonic vibrating component or sheet-shaped piezoelectric ceramics, or may be, for example, an ultrasonic atomizer tablet provided in the patent No. CN112335933A, or the like. In use, such a vibrable element 12 disperses the liquid substrate through high-frequency vibration (a preferable vibration frequency ranges from 1.7 MHz to 4.0 MHz, which is beyond the human hearing range and belongs to an ultrasonic frequency band) to produce an aerosol whose particles are naturally suspended.

In an optional implementation, the liquid delivery element 13 may be a common capillary element, for example, cellucotton or a porous body. In another preferable implementation, the liquid delivery element 13 may be a micropump, which pumps a predetermined amount of the liquid substrate from the liquid storage chamber 11 to the vibrable element 12, for example, a micropump based on the microelectromechanical systems (MEMS) technology. Examples of suitable micropumps include the model MDP2205 micropump and other micropumps of thinXXS Microtechnology AG, the model mp5 and mp6 micropumps and other micropumps of Bartels Mikrotechnik GmbH, and the piezoelectric micropumps of Takasago Fluidic Systems.

Further, the circuit 22 includes several electronic components, and in some examples, may be formed on a printed circuit board (PCB) that supports and electrically connects electronic components. The electronic component may include a microprocessor or a processor core and a memory. In some examples, the control component may include a microcontroller having a processor core and memory that are integrated, and may further include one or more integrated input/output peripherals.

According to FIG. 2, the circuit 22 includes:
an MCU controller 221; and
a drive module 222, positioned between the cell 10 and the vibrable element 12 and configured to guide a direct current between them. Based on the common implementation of ultrasonic atomization, a current that drives the vibrable element 12 to perform ultrasonic atomization may be of two types, one is a common high-frequency AC, and the other is a pulse-type current with a constant direction and a changing magnitude. Specifically, in a preferable implementation, the drive module 222 preferably generates, through guidance, an alternating current including a positive current and a negative current that are periodic, and supplies the alternating current to the vibrable element 12.
a peak voltage detection module 223, configured to detect a peak voltage at both terminals of the vibrable element 12; and

Further, FIG. 3 shows a schematic structural diagram of basic devices of the drive module 222 in an embodiment. In the embodiment shown in FIG. 3, the drive module 222 includes:
a first inductor L1, having a first terminal connected to a voltage output terminal of the cell 21 and a second terminal connected to a first terminal of the vibrable element 12;
a second inductor L2, having a first terminal connected to the voltage output terminal of the cell 21 and a second terminal connected to a second terminal of the vibrable element 12;
a first switching tube Q1, having a D pole connected to the second terminal of the first inductor L1, an S pole grounded, and a G pole receiving a pwm1 driving signal from the MCU controller 221, to further switch on/off; and
a first switching tube Q2, having a D pole connected to the second terminal of the second inductor L2, an S pole grounded, and a G pole receiving a pwm2 driving signal from the MCU controller 221, to further switch on/off.

The foregoing drive module 222 periodically guides and supplies the alternating positive and negative currents to the vibrable element 12 through alternating switch-on of the first switching tube Q1 and the second switching tube Q2, to enable the vibrable element 12 to vibrate in a reciprocating manner.

Further, for a working state of the vibrable element 12, the following cases are included:
If no liquid substrate is provided to the vibrable element 12, a vibration process of the vibrable element 12 may be equivalent to vibration without damping. If a liquid substrate is provided to the vibrable element 12, a vibration process of the vibrable element 12 may be equivalent to damped vibration. Therefore, an amplitude of the vibrable element 12 during vibration and the peak voltage at the both terminals of the vibrable element 12 are related to a driving frequency, a driving current, and a damping force that the vibrable element 12 is subjected to.

The driving frequency and the driving current are set by the MCU controller 221 and may be considered as being given or known. Then, the peak voltage is basically only related to the damping, and the damping force is provided and generated by the liquid substrate. Based on this, whether the liquid substrate on the vibrable element 12 is adequate can be determined by detecting the peak voltage, and further, whether "dry burning" occurs can be determined.

In a further preferable implementation, to improve the vibration efficiency of the vibrable element 12, during the control performed by the MCU controller 221, the driving frequency is enabled to be the same as or very close to a natural vibration frequency of the vibrable element 12, so that the vibrable element 12 is in a resonance state during operation, and has the maximum efficiency. Similarly, the amplitude and the peak voltage are also the largest in the resonance state. Furthermore, in another preferable implementation, the MCU controller 221 may also find or obtain an optimal resonant frequency of the vibrable element 12 through detection on the peak voltage in the foregoing resonance state, and use the optimal resonant frequency as the driving frequency to drive the vibrable element 12 to work.

Further, FIG. 4 shows a schematic diagram of a positive voltage signal S1 of the vibrable element 12 and a PWM1 control signal S2 sampled at a first sampling point W1 in FIG. 3. Likewise, it can be learned that a voltage change sampled at a second sampling point W2 and the positive voltage signal S1 sampled at the first sampling point W1 jointly form a voltage change at both terminals of the vibrable element 12, which is generally in a simple harmonic waveform.

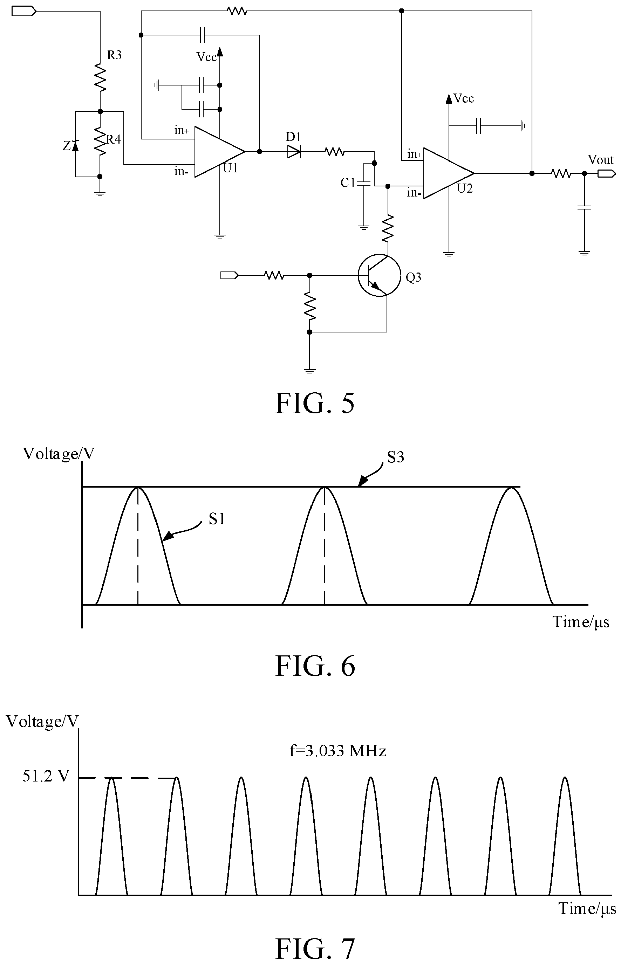

Further, FIG. 5 shows a schematic structural diagram of basic devices of the peak voltage detection module 223 in an embodiment. The peak voltage detection module 223 includes: an operational amplifier U1, a holding capacitor C1, and a voltage follower U2.

A sampling terminal in- of the operational amplifier U1 is connected to the first sampling point W1 and/or the second sampling point W2 at the both terminals of the vibrable element 12 in FIG. 3, to obtain a voltage at the both terminals of the vibrable element 12 and output an operation result through a diode D1.

The holding capacitor C1 is connected to an output terminal of the operational amplifier U1, so that an output peak voltage of the operational amplifier U1 can be further held or locked through the holding capacitor C1. For example, in a time period of t1 to t2 in a voltage change cycle shown in FIG. 4, the operational amplifier U1 outputs a voltage whose value gradually increases, and the holding capacitor C1 receives the output voltage and stores the voltage until the moment t2 at which the value of the voltage output by the operational amplifier U1 reaches a maximum, where at this moment, a voltage at both terminals of the holding capacitor C1 reaches a maximum synchronously. Further, after the moment t2 is passed, the output of the operational amplifier U1 gradually decreases until it is 0. However, because the holding capacitor C1 does not discharge, a value of the voltage at the both terminals of the holding capacitor C1 is always held at a peak.

The voltage follower U2 follows to output the peak voltage held by the holding capacitor C1.

Through the foregoing holding capacitor C1 and the foregoing voltage follower U2, the output peak voltage can always be held at any time during detection, and further the MCU controller 221 can obtain or sample the detected peak voltage at any time.

In the preferable implementation shown in FIG. 5, the operational amplifier U1 is used as a comparator, which is a basic use. Specifically, if a reference signal input terminal in+ of the operational amplifier U1 is connected to a constant signal in output signals by a capacitor, the operational amplifier U1, as a comparator, outputs a result of a comparison operation between the voltage at the both terminals of the vibrable element 12 and a constant reference signal. That is, when a voltage signal sampled at a sampling terminal in- of the operational amplifier U1 is higher than a reference voltage signal input at the input terminal in+, the operational amplifier U1 outputs a comparison result to the holding capacitor C1 for holding until the voltage signal sampled at the sampling terminal in- is a peak, where at this moment, a maximum voltage received by the holding capacitor C1 is a peak voltage.

Specifically, FIG. 6 shows a schematic diagram of a voltage signal of a result signal Vout output by the peak voltage detection module 223. It can be learned from FIG. 6, a signal S3 output by the peak voltage detection module 223 is always a peak voltage at the both terminals of the vibrable element 12.

Further, in a preferable implementation shown in FIG. 5, based on a conventional follow-to-output connection manner, a sampling terminal in+ of the voltage follower U2 is connected to a sampling terminal in+ of the operational amplifier U1. In addition, the peak voltage detection module 223 further includes several basic components such as a resistor and a capacitor, which are configured for basic functions, that is, voltage dividing, voltage stabilization, and current limiting.

Specifically, in the implementation shown in FIG. 5, a negative terminal of the holding capacitor C1 in the peak voltage detection module 223 is grounded, and connections of a positive terminal of the holding capacitor C1 include three channels:
The first channel is that the positive terminal of the holding capacitor C1 is connected to an output terminal of the operational amplifier U1, and is configured to receive a voltage output by the operational amplifier U1.

The second channel is that the positive terminal of the holding capacitor C1 is connected to a sampling terminal in- of the voltage follower U2, to enable the voltage follower U2 to output the peak voltage held by the holding capacitor C1.

The third channel is that the positive terminal of the holding capacitor C1 is grounded through a switching tube Q3, and the MCU controller 221 discharges the positive terminal of the holding capacitor C1 to 0 by switching on the switching tube Q3, to facilitate sampling of a next peak voltage.

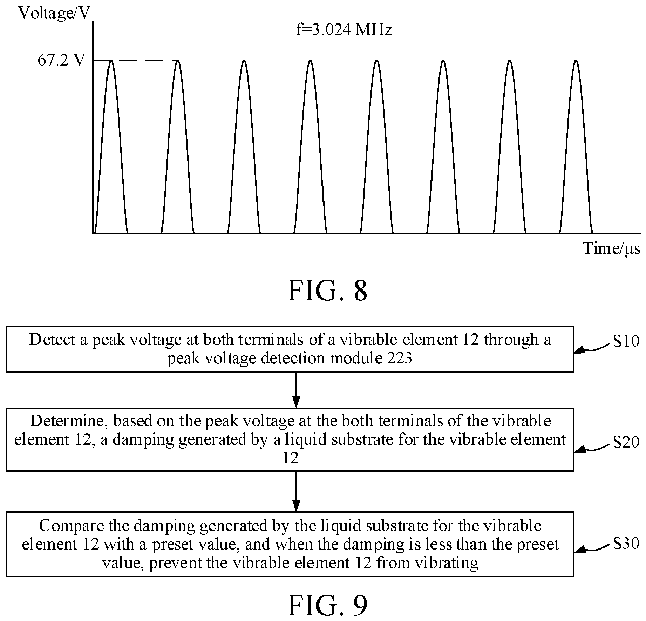

FIG. 7 shows a schematic diagram of a voltage change at both terminals of the vibrable element 12 when the liquid substrate in the liquid storage chamber 11 of the electronic atomization apparatus is adequate in an embodiment. FIG. 8 shows a schematic diagram of a voltage change at both terminals of the vibrable element 12 after the liquid substrate in the liquid storage chamber 11 of the electronic atomization apparatus is consumed completely in an embodiment. It can be learned from FIG. 7 and FIG. 8 that at substantially same driving frequencies and substantially same driving currents, the peak voltage at the both terminals of the vibrable element 12 during operation is 51.2 V when the liquid substrate is inadequately supplied, while the peak voltage at the both terminals of the vibrable element 12 is 67.2 V during operation when no liquid substrate is supplied. Further, based on a change in the peak voltage of the vibrable element 12 caused by the damping provided by the liquid substrate, whether the liquid substrate is adequately supplied can be determined, and further, whether "dry burning" occurs can be determined.

Further, an embodiment of this application provides a method of determining, by detecting a damping of the liquid substrate in the vibrable element 12, whether supply of the liquid substrate is adequate and the like and further controlling the electronic atomization apparatus, which, as shown in FIG. 9, includes the following steps:
S 10. A peak voltage detection module 223 detects a peak voltage at both terminals of the vibrable element 12.
S20. Determine, based on the peak voltage at the both terminals of the vibrable element 12, a damping generated by a liquid substrate for the vibrable element 12.
S30. Compare the damping generated by the liquid substrate for the vibrable element 12 with a preset value, and when the damping is less than the preset value, prevent the vibrable element 12 from vibrating.

In the foregoing steps, whether the liquid substrate is adequately supplied to the vibrable element 12 is further monitored based on a difference in the peak voltage at the both terminals of the vibrable element 12 caused by the amount of the liquid substrate supplied to the vibrable element 12 during vibration. When the damping is lower than the preset value and is too small, it indicates that the amount of the liquid substrate on the vibrable element 12 is inadequate, and the controller 221 can perform control to prevent the vibrable element 12 from continuing vibrating, to avoid "dry burning".

The foregoing method may also be used to determine whether the liquid substrate carried on the vibrable element 12 is completed atomized in one quantity of times of vaping. In one quantity of times of vaping, when the liquid substrate is gradually reduced by atomization, the damping is gradually reduced, and the peak voltage of the vibrable element 12 is gradually increased. Furthermore, in one quantity of times of vaping, when the damping decreases to be less than the preset value, it indicates that the liquid substrate carried on the vibrable element 12 has been almost atomized, so that the vibrable element 12 can be controlled to stop vibrating.

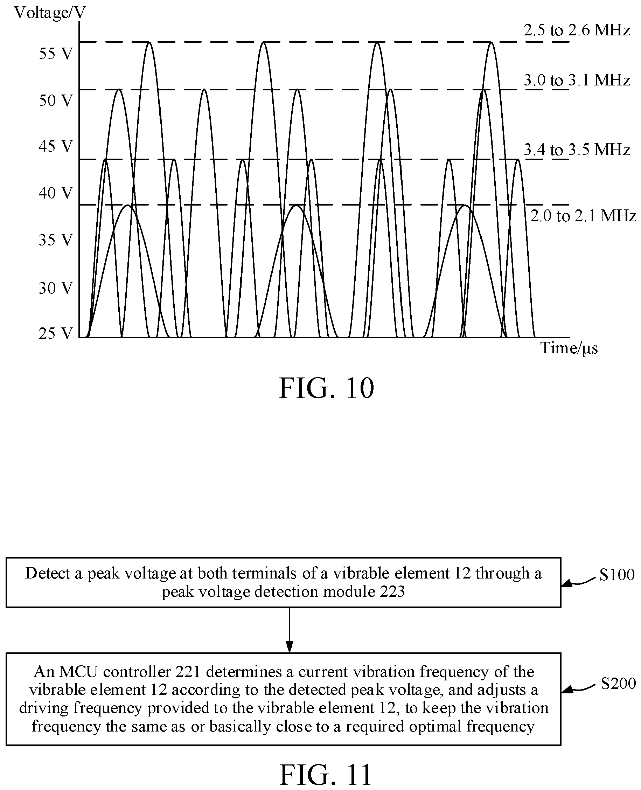

Further, in another preferable implementation, FIG. 10 shows a result of obtaining an optimal driving frequency of the vibrable element 12 through a frequency sweep. The vibrable element 12 is driven respectively through series of different frequencies (which may be from high to low or from low to high) sent by the MCU controller 221, to work, and the peak voltage is detected correspondingly by the foregoing peak voltage detection module 223. It can be learned from FIG. 9 that the peak voltage differs at different driving frequencies. In addition, in one of the frequency bands, that is, 2.5 to 2.6 MHz, the peak voltage may reach a maximum. In this case, it can be basically determined that a frequency corresponding to the maximum peak voltage is a natural frequency of the vibrable element 12. Driving the vibrable element 12 at the natural frequency enables the vibrable element 12 to work at the optimal resonance.

The foregoing "frequency sweep" is an electrical term, which refers to using a signal to continuously change from a high frequency to a low frequency (or from a low frequency to a high frequency) within a frequency band, which is usually for testing.

Based on the above, another embodiment of this application further provides a method of automatically seeking and obtaining an optimal vibration frequency, including: driving, through a frequency sweep method, the vibrable element 12 to vibrate, and detecting a peak voltage at both terminals of the vibrable element 12; and determining, when the peak voltage at the both terminals of the vibrable element 12 reaches a maximum, that a frequency of a current frequency sweep is a resonant frequency that is sought.

Based on the above, another embodiment of this application further provides a method of adaptively adjusting a vibration frequency of the vibrable element 12 of the electronic atomization apparatus, which, as shown in FIG. 11, includes the following steps:
S100. Detect a peak voltage at both terminals of the vibrable element 12 through a peak voltage detection module 223.
S200. An MCU controller 221 determines a current vibration frequency of the vibrable element 12 according to the detected peak voltage, and adjusts a driving frequency provided to the vibrable element 12, to keep the vibration frequency the same as or basically close to a required optimal frequency.

In this embodiment, a correlation between the peak voltage detected by the peak voltage detection module 223 and the frequency is used to reversely calculate the current vibration frequency, and then, the driving frequency is adaptively adjusted to be the same as or basically close to the optimal resonant frequency.

It should be noted that the specification and the drawings of this application provide preferred embodiments of this application, but are not limited to the embodiments described in this specification. Further, a person of ordinary skill in the art may make improvements or modifications according to the foregoing description, and all of the improvements and modifications should all fall within the protection scope of the attached claims of this application.

## Claims

1. An electronic atomization apparatus, comprising:
a liquid storage chamber, configured to store a liquid substrate;
a vibrable element, configured to generate vibration to atomize the liquid substrate for generating an aerosol;
a peak voltage detection module, configured to detect a peak voltage at both terminals of the vibrable element; and
a controller, configured to control vibration of the vibrable element based on the peak voltage.

2. The electronic atomization apparatus according to claim 1, wherein the controller is configured to determine, based on the peak voltage, a damping that the vibrable element is subjected to, and control vibration of the vibrable element according to the damping.

3. The electronic atomization apparatus according to claim 2, wherein the controller is configured to compare the damping with a preset value, and prevent, when the damping is less than the preset value, the vibrable element from vibrating.

4. The electronic atomization apparatus according to any one of claims 1 to 3, wherein the peak voltage detection module comprises:
a holding capacitor, configured to hold the peak voltage at the both terminals of the vibrable element.

5. The electronic atomization apparatus according to claim 4, wherein the peak voltage detection module further comprises:
an operational amplifier, configured to output a voltage at the both terminals of the vibrable element to the holding capacitor; and
a voltage follower, configured to output the peak voltage that are at the both terminals of the vibrable element and that are held by the holding capacitor.

6. The electronic atomization apparatus according to claim 5, wherein the peak voltage detection module further comprises:
a discharge switch, configured to discharge the holding capacitor in an on state.

7. The electronic atomization apparatus according to claim 6, wherein a sampling terminal of the operational amplifier is connected to the vibrable element; and
the holding capacitor comprises three channels, wherein a first channel is connected to an output terminal of the operational amplifier, a second channel is connected to the discharge switch, and a third channel is connected to a sampling terminal of the voltage follower.

8. The electronic atomization apparatus according to any one of claims 1 to 3, wherein the vibrable element comprises at least piezoelectric ceramics.

9. The electronic atomization apparatus according to any one of claims 1 to 3, wherein the controller is configured to determine a vibration frequency of the vibrable element based on the peak voltage.

10. The electronic atomization apparatus according to claim 9, wherein the controller is further configured to:
adjust the vibration frequency of the vibrable element, to keep the vibration frequency the same as or substantially close to a preset frequency.

11. The electronic atomization apparatus according to any one of claims 1 to 3, wherein the controller is configured to:
use a changing frequency to drive the vibrable element to vibrate, and detect the peak voltage at the both terminals of the vibrable element; and
determining an optimal resonant frequency of the vibrable element according to a maximum of the peak voltage.

12. An electronic atomization apparatus, comprising:
a liquid storage chamber, configured to store a liquid substrate;
a vibrable element, configured to generate vibration to atomize the liquid substrate for generating an aerosol; and
the controller, configured to control vibration of the vibrable element according to a damping that the vibrable element is subjected to.

13. The electronic atomization apparatus according to claim 12, wherein the controller is configured to compare the damping with a preset value, and prevent, when the damping is less than the preset value, the vibrable element from vibrating.

14. A control method for an electronic atomization apparatus, the electronic atomization apparatus comprising a liquid storage chamber, configured to store a liquid substrate; and
a vibrable element, configured to generate vibration to atomize the liquid substrate for generating an aerosol, wherein
the method comprises:
detecting a peak voltage at both terminals of the vibrable element; and
controlling vibration of the vibrable element based on the peak voltage.

15. The control method for an electronic atomization apparatus according to claim 14, wherein the controlling vibration of the vibrable element based on the peak voltage comprises:
determining, based on the peak voltage, a damping that the vibrable element is subjected to, and controlling vibration of the vibrable element according to the damping.

16. The control method for an electronic atomization apparatus according to claim 14, wherein the controlling vibration of the vibrable element based on the peak voltage comprises:
determining a vibration frequency of the vibrable element based on the peak voltage; and
adjusting the vibration frequency of the vibrable element, to keep the vibration frequency the same as or substantially close to a preset frequency.
